(19) Europäisches Patentamt
European Patent Office
Office européen des brevets

(11) Publication number: 0 097 451
A1

(12) # EUROPEAN PATENT APPLICATION

(21) Application number: 83303322.8

(22) Date of filing: 08.06.83

(51) Int. Cl.³: C 07 F 9/65
C 07 D 239/30, A 01 N 57/08

(30) Priority: 18.06.82 US 389638

(43) Date of publication of application:
04.01.84 Bulletin 84/1

(84) Designated Contracting States:
BE CH DE FR GB IT LI NL SE

(71) Applicant: THE DOW CHEMICAL COMPANY
Dow Center 2030 Abbott Road Post Office Box 1967
Midland Michigan 48640(US)

(72) Inventor: Reifschneider, Walter
3538 Bayberry Drive
Walnut Creek California(US)

(74) Representative: Allard, Susan Joyce et al,
BOULT, WADE & TENNANT 27 Furnival street
London EC4A 1PQ(GB)

(54) **2-Alkyl-5-pyrimidines.**

(57) Novel 2-alkyl-5-halopyrimidines, e.g., 5-chloro-2-(1,1-dimethylethyl)pyrimidine, which are useful as intermediates in the preparation of, e.g., 0,0-diethyl 0-(2-(1,1-dimethylethyl)-5-pyrimidinyl) phosphorothioate which is useful as an insecticide against corn rootworm and cutworm.

EP 0 097 451 A1

0097451

# 2-ALKYL-5-HALOPYRIMIDINES

This invention relates to novel 2-alkyl--5-halopyrimidines. The invention provides a process for producing them and their use as intermediates in making insecticides useful for the control of insects such as corn rootworm and cutworm.

The preparation of 5-halogenopyrimidine is described in "Heterocyclic Compounds, The Pyrimidines, Supplement I" (1970) Wiley-Interscience, pages 119 ff. The compounds 5-chloro- and 5-bromo-2-methylpyrimidine and 5-chloro- and 5-bromopyrimidine are known (CA 44, 1516e), as well as the compounds 5-chloro-4-tert-butylpyrimidine and 5-bromo-4-tert-butylpyrimidine (CA 90, 72140b and 83, 42517t).

The novel compounds of this invention are 2-alkyl-5-halopyrimidines having the formula:

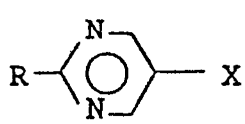

wherein R is cyclopropyl, isopropyl or t-butyl and X is Cl or Br.

Certain of these compounds have biocidal activity and the may be employed, for example, in the control of mildew and rice blast. In addition, these compounds are advantageously employed as intermediates in the preparation of O-alkyl-O-[pyrimidin(5)yl]-(thiono)(thiol)-phosphoric (phosphonic) acid esters or ester-amides having exceptional insecticidal activity by the processes described in, for example, U.S. Patent 4,127,652. For such processes, the halopyrimidines are first hydrolyzed as taught, for example, in Supplement I of "The Pyrimidines", Interscience (1970), page 148 or in U.S. Patent 4,379,930.

Accordingly, the invention also provides a process for making a compound having the formula

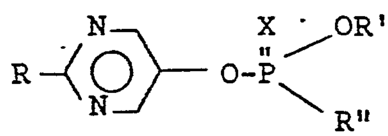

wherein R is cyclopropyl, isopropyl or t-butyl; R' is alkyl having 1 to 4 carbon atoms; X is oxygen or sulfur; and R" is alkyl of 1 to 2 carbon atoms; alkoxy, alkylthio or monoalkylamino, each with 1 to 4 carbon atoms, or phenyl, which comprises the steps of (A) hydrolyzing a compound having the formula

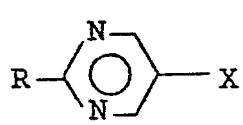

wherein R is as above defined and X is Cl or Br, to obtain a compound having the formula

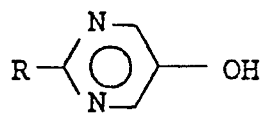

wherein R is as above defined, and (B) reacting the resulting pyrimidinol with the desired phosphorus chloride in the presence of an acid acceptor and an inert solvent at a temperature of 0°-100°C.

Preferred phosphorus chlorides are O,O-diethyl phosphorochloridothioate and O-ethyl (1-methylethyl) phosphorochloridothioate.

The hydrolysis of the 2-alkyl-5-halopyrimidine (step A) is advantageously carried out in the presence of an alkali metal methoxide and a catalytic amount of an N-oxide, a disulfide or elemental sulfur. A preferred N-oxide is 2-picoline-N-oxide. A preferred disulfide is di-n-butyl-disulfide.

The hydrolysis reaction is advantageously carried out at temperatures of from 100° to 300°C, preferably 150° to 220°C and most advantageously at 160° to 180°C at ambient pressure in a Parr bomb, in the presence of from 0.5 to 20 mole percent of catalyst. The preferred catalyst is elemental sulfur and it is preferably employed in an amount of from 2.5 to 7.5 mole percent. Sodium methoxide is preferably employed, although other alkali metal methoxides such as potassium methoxide may be employed, if desired.

The hydrolysis is advantageously and preferably carried out in a methyl alcohol solvent.

The reaction of step B may be carried out conveniently in any inert organic liquid such as benzene, toluene, xylene, chlorobenzene, petroleum ether, methylene chloride, chloroform, carbon tetrachloride, ethers, such as diethyl ether, dibutyl ether and dioxane, ketones, such as acetone, methyl ethyl ketone, methyl isopropyl ketone or methyl isobutyl ketone and nitriles, such as acetonitrile and propionitrile.

Also employed is an acid binding agent or acceptor selected from alkali carbonates, alkali hydroxides, and alcoholates such as sodium carbonate, potassium carbonate, sodium or potassium methylate or ethylate and aliphatic, aromatic or heterocyclic amines, for example, triethylamine, dimethylaniline, dimethylbenzylamine and pyridine.

The reaction temperature can be selected from a rather large range between 0° to 100°C, preferably 20° to 60°C. The reaction is generally carried out under ambient atmospheric pressure conditions.

The amounts of the reagents to be employed are not critical, some of the desired products being obtained when employing any proportion of the reactants. In the preferred method of operation, good results are obtained when employing substantially equimolecular proportions of the pyrimidinol and phosphorochloridate, phosphoroamidochloridothioate, phosphonochloridothioate or phosphorochloridothioate. The reaction takes place smoothly at the temperature range from 0° to 100°C with the production of the desired product and chloride by-product. In carrying out the reaction, the

reactants are mixed and contacted together in any convenient fashion, and the resulting mixture maintained for a period of time in the reaction temperature range to complete the reaction. Following the completion of the reaction, the reaction mixture is washed with water and any organic reaction medium removed by distillation under reduced pressure to obtain the desired product as a residue. This product can be further purified by conventional procedures such as washing with water and dilute aqueous alkali metal hydroxide, solvent extraction and recrystallization.

The invention is further illustrated by the following examples.

Example 1 - 5-Chloro-2-cyclopropylpyrimidine

To a stirred mixture of 15.5 g of cyclopropanecarboximidamide monohydrochloride, 30.9 g of N-(2-chloro-3-(dimethylamino)2-propylidene)-N-methylmethanaminium perchlorate and 50 ml of methanol was added dropwise, a sodium methoxide solution prepared from 8.2 g of sodium and 150 ml of methanol. After the addition was complete, the mixture was heated under reflux for approximately three hours. The reaction mixture was concentrated under vacuum and the distillate which contained some product was saved. The residue was taken up in ether, washed with water, saturated in sodium chloride solution and dried over anhydrous sodium sulfate. The ether solution was combined with the distillate and the solvents were removed by distillation using a 50 cm vacuum jacketed Vigreux column leaving 16 g (87 percent of theoretical) of product. The material was distilled in a Kugelrohr

(bath temperature 45°C, pressure 0.1 mm) to give a colorless oil which solidified on standing. Recrystallization from hexane gave white crystals, m.p. 37°-38°C.
Analysis: Found: C, 54.10; H, 4.88; N, 18.25.
Calculated for $C_7H_7ClN_2$: C, 54.38; H, 4.56; N, 18.12.

Example 2 - 5-Chloro-2-(1-methylethyl)pyrimidine)

A Vilsmeier reagent was prepared below 0°C from 610 ml of dimethylformamide and 190 g of phosgene and 60 g of chloroacetic acid were added. The mixture was then carefully warmed to 70°C, kept at this temperature for two hours and was then heated to 85°C for another two hours. The excess dimethylformamide was removed as far as practical under vacuum. The residue was taken up in 300 ml of methanol, 70 g of 2-methylpropanimidamide monohydrochloride was added and to the resulting mixture 900 ml of 25 percent sodium methoxide in methanol were added dropwise. After the addition was complete, the mixture was heated under reflux for three hours. The salts were removed by filtration and the filtrate concentrated under vacuum. The residual oil was triturated with water and the mixture extracted several times with ether. The ether extract was washed with saturated sodium chloride solution and dried over anhydrous sodium sulfate. The ether was removed under vacuum and the residual oil fractionated on a small Vigreux column, yielding 20 g (22 percent of theoretical) of a colorless oil RI (25°C) = 1.4930; b.p. 80°C at 12 mm.
Analysis: Found: C, 53.58; H, 6.02; N, 17.74.
Calculated for $C_7H_9ClN_2$: C, 53.68; H, 5.79; N, 17.89.

Example 3 - 5-Chloro-2-(1,1-dimethylethyl)pyrimidine

To a stirred mixture of 80 g of N-(2-chloro--3-(dimethylamino)-2-propylidene)-N-methylmethanaminium perchlorate, 46 g of 2,2-dimethylpropanimidamide monohydrochloride and 100 ml of methanol was added dropwise a sodium methoxide solution, prepared from 21 g of sodium and 250 ml of methanol. After the addition was complete, the mixture was heated under reflux for two hours. The reaction mixture was concentrated under vacuum and the distillate which contained some product was saved. The residue was taken up in ether, washed twice with water, once with saturated sodium chloride solution and dried over anhydrous sodium sulfate. The ether extract was combined with the distillate and the solvents were removed by distillation, using a Vigreux column. The residue was then fractionated in a small Vigreux column and 42 g (80 percent of theoretical) of colorless oil were collected at 82°C and 18 mm RI (25°C) = 1.4944. The oil solidified on standing.

Analysis: Found: C, 56.46; H, 6.49; N, 16.46.
Calculated for $C_8H_{11}ClN_2$: C, 56.30; H, 6.50; N, 16.42.

Example 4 - 5-Bromo-2-(1,1-dimethylethyl)pyrimidine

A mixture of 54.5 g of 2-(1,1-dimethylethyl)-pyrimidine, 100 g of potassium acetate and 250 ml of glacial acetic acid were heated to gentle reflux and bromine was added dropwise until GLC-analysis (Gas Liquid Chromatographic analysis) showed only traces of starting material. For this 110 g of bromine was needed (theoretical 64 g). The reaction mixture was carefully concentrated under vacuum (reaction product is volatile), the residue dissolved in water and made alkaline by the addition of 20 percent aqueous

sodium hydroxide. The product precipitated and was extracted into ether. The ether solution was washed once with saturated sodium chloride solution and dried over anhydrous sodium sulfate. The solvent was removed in a rotary evaporator leaving 83.5 g (97 percent of theoretical) of a white solid. Purity by GLC was 98 percent. Recrystallization from hexane yielded white crystals, m.p. 50°-52°C. Analysis: Found: C, 44.11; H, 4.92; N, 12.64. Calculated for $C_8H_{11}BrN_2$: C, 44.67; H, 5.16; N, 13.03.

The compounds 2-cyclopropyl-5-bromo-pyrimidine and 2-isopropyl-5-bromo-pyrimidine are readily prepared as described in Example 3 except for using N-(2-bromo-3-(dimethylamino)-2-propylidene-N-methylmethanaminium perchlorate and the appropriate imidamide as starting materials.

CLAIMS:

1. A compound having the formula:

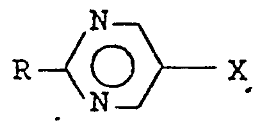

wherein R is a cyclopropyl, isopropyl or t-butyl group and X is a chlorine or bromine atom.

2. 5-Chloro-2-cyclopropylpyrimidine.

3. 5-Chloro-2-(1-methylethyl)pyrimidine.

4. 5-Chloro-2-(1,1-dimethylethyl)pyrimidine.

5. 5-Bromo-2-(1,1-dimethylethyl)pyrimidine.

6. A process for the preparation of a compound of the formula:

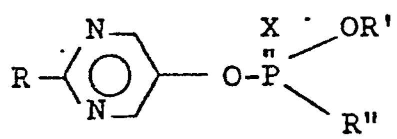

wherein R is a cyclopropyl, isopropyl or t-butyl group; R is an alkyl group containing 1 to 4 carbon atoms; X is oxygen or sulfur; and R" is an alkyl group containing 1 or 2 carbon atoms, an alkoxy, alkylthio or monoalkylamino group each containing 1 to 4 carbon atoms, or a phenyl group, which process comprises the steps of

(a) hydrolyzing a compound of the formula:

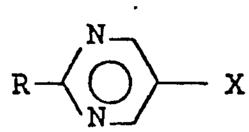

wherein R is as above defined and X is a chlorine or bromine atom to obtain a compound having the formula:

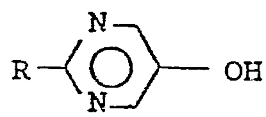

wherein R is as above defined, and

(b) reacting the resulting pyrimidinol with the desired phosphorus chloride in the presence of an acid acceptor and an inert solvent at a temperature in the range of from $0^{o}$ to $100^{o}C$.

7. A process as claimed in claim 6 wherein the phosphorus chloride is O,O-diethylphosphorochloridothioate or O-ethyl(1-methylethyl)phosphorochloridothioate.

8. A process as claimed in claim 6 or claim 7 wherein the hydrolysis is carried out in the presence of an alkali metal methoxide and a catalytic amount of an N-oxide, a disulfide or elemental sulfur.

European Patent Office

# EUROPEAN SEARCH REPORT

0097451
Application number

EP 83 30 3322

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,A | CHEMICAL ABSTRACTS, vol. 44, 1951, abstract no. 1516e, Columbus, Ohio, USA Z. BUDESINSKY: "Derivatives of 2-methylpyrimidine" & COLLECTION CZECHSLOV. CHEM. COMMUNS. 14,223-35(1949) * Column 2, paragraphs e-g * | 1 | C 07 F 9/65<br>C 07 D 239/30<br>A 01 N 57/08 |
| D,Y | D.J. BROWN: "The Pyrimidines Supplement I", 1970, Chapter VI E, Wiley-Interscience, New York, USA * Pages 148-149 * | 6 | |
| Y | EP-A-0 009 566 (BAYER) * Pages 1-6; claims * | 6,7 | |
| D,Y | FR-A-2 365 577 (BAYER) * Pages 1-5; claims * | 6,7 | |

TECHNICAL FIELDS SEARCHED (Int. Cl. 3)

C 07 F 9/00
C 07 D 239/00

The present search report has been drawn up for all claims

| Place of search | Date of completion of the search | Examiner |
|---|---|---|
| THE HAGUE | 30-09-1983 | FRANCOIS J.C.L. |

CATEGORY OF CITED DOCUMENTS

X : particularly relevant if taken alone
Y : particularly relevant if combined with another *document of the same category*
A : technological background
O : non-written disclosure
P : intermediate document
T : theory or principle underlying the invention
E : earlier patent document, but published on, or after the filing date
D : document cited in the application
L : *document cited for other reasons*
& : member of the same patent family, corresponding document